# EUROPEAN PATENT SPECIFICATION

(11) **EP 3 697 291 B1**
(45) Date of publication and mention of the grant of the patent: **19.11.2025**
(21) Application number: 19739287.1
(22) Date of filing: 12.07.2019
(51) Int. Cl.: A61B 5/00, A61B 5/021, A61B 5/022, A61B 17/135

(54) **WEARABLE BLOOD PRESSURE MEASUREMENT DEVICE AND METHOD**
AM KÖRPER TRAGBARES BLUTDRUCKMESSGERÄT UND METHODE
DISPOSITIF PORTABLE DE MESURE DE LA PRESSION ARTÉRIELLE ET PROCÉDÉ

(30) Priority: 16.07.2018 IT 201800007236
(43) Date of publication of application: 26.08.2020
(73) Proprietor: Scardulla, Francesco, 90139 Palermo (PA) (IT)
(72) Inventor: Scardulla, Francesco, 90139 Palermo (PA) (IT)
(74) Representative: De Tullio, Michele Elio
(86) International application number: PCT/EP2019/068907
(87) International publication number: WO 2020/016139

(56) References cited:
- US-A1- 2008 139 949
- US-A1- 2010 106 029
- US-A1- 2011 009 757
- US-A1- 2015 286 277
- US-A1- 2017 319 136

## Description

### Field of the invention

The present invention concerns an arterial blood pressure measuring device and method.

### Background of the invention

With the recent advances in microelectronics, telecommunications, sensor manufacturing and data analysis techniques, wearable sensor technologies have emerged for remote monitoring of vital signs and other health factors such as blood pressure, heart rate, heart rate variability, sweat, body temperature, movement and blood oxygenation. This clinical information can be used to monitor and prevent numerous diseases as well as inappropriate behaviours.

Blood pressure (BP) is one of the most basic vital signs and provides physicians and users with important metrics regarding the patient's baseline cardiovascular status in both clinical and sports applications.

Photoplethysmography (PPG) enables the gathering of information related to volume variations and light absorption within the cardiovascular system and, consequently, to cardiac activity, such as heartbeat, blood oxygenation and heart rate variability. Small sensors based on photoplethysmography technology have been developed to measure BP. These devices comprise light-emitting diodes (LEDs) and photodetectors and allow the monitoring of the pulse rate in a non-invasive manner. For example, the patent publication no. US 2017/0251935 describes a wearable watch having a PPG sensor to measure blood pressure.

US2011/0009757 A1 teaches a sphygmomanometer that tightens an air cuff before the injection of air to make easier the positioning and the fastening of the air cuff on an arm of a user. The system increases the stability and precision in blood pressure measurements and makes it easier to wear the air cuff before the measurement is carried out. The device described this '757 patent document does not use any type of PPG sensor and measures the blood pressure in a traditional way (i.e. by measuring in the internal pressure of fluid bag). This requires therefore the mandatory presence of an air bladder in the device. The tightening system (i.e. the winding roller) of the device is used to properly fit the cuff before the blood pressure measurement and not to measure the blood pressure.

US2017/0319136 A1 teaches a sensor platform which is moved by an actuator with respect to the skin. The sensor platform is configured to adjust the position towards and away from the skin. The sensor platform receives the signals from different sensors (e.g. PPG sensor) and then the sensor platform adjusts the position of the sensor platform (with respect of the skin) to increase the quality of the signal because of the need to increase contact pressure between the sensor and the skin or to compensate movements of forces. The sensor platform does not teach the measurement of arterial blood pressure and cannot be used to apply a tourniquet.

Similarly, US patent no 9,781,984 B2 teaches a system and method for dynamically adjusting the fit of a wearable device. The system can be used to increase the accuracy of measurements performed by a PPG sensor by adapting the wearable device to fit more snugly to the user. The system does not disclose a methodology to measure blood pressure, nor can the dynamic tightening system described in the document be used as a tourniquet.

A tightening system to perform ischemic preconditioning is disclosed in US Patent Application No. US2008/0139949 A1. The tightening system can be used with a PPG sensor, but it is not a blood pressure measurement system and cannot be used as a tourniquet to prevent bleeding in case of injuries. The PPG sensors are used only to identify reperfusion (restoration of blood) after a tightening period

Another device is known from US Patent No. 9,301,701 B2 which teaches a method of for determining an occlusion pressure of a limb. The device cannot be used as an emergency tourniquet.

Other patent documents include US 2018/0153418 A1 Jun 2018 which teaches a blood pressure wearable device using a tightening system with one or more pressure sensors, but not a PPG sensor. US 2015/0286277 A1 teaches a method includes detecting, by a pressure sensor, a level of pressure applied by the body-wearable device to the user, but is not a blood pressure measurement system but a system to control the tightening level. US2010/106029 A1 relates to methods and medical apparatuses for non-invasive monitoring of arterial blood pressure, and specifically to the devices and methods that use inflatable cuffs.

Several other techniques have been developed in order to measure blood pressure by using PPG sensors; most of them suffer from a poor accuracy, applicability and reliability.

It is therefore an object of the present disclosure to provide an improved wearable blood pressure device and a method for measuring blood pressure.

It is another object of the present invention to provide a device that, in a different configuration, is capable of blocking blood haemorrhage, even in adverse conditions, using a tourniquet.

### Summary of the invention

The present invention proposes a wearable arterial blood pressure measuring device for measuring the systolic pressure and the diastolic pressure of a user according to claim 1. The blood pressure measuring device includes a tightening device comprising a tightening system adapted to tighten the tightening device on or around the region of interest, wherein the tightening system is adapted to be automatically adjusted in one of a plurality of fastening positions, and one of a processor or control circuitry to control the tightening system. A blood sensor, such as but not limited to a PPG sensor, a piezoelectric sensor or an ultrasound sensor, is placed on at least one side of the tightening system. In the claimed invention, the blood sensor is a PPG sensor outputting a PPG signal.

The device comprises a pressure sensor for measuring the tightening pressure exerted by the tightening system on the region of interest, in particular wherein the pressure sensor is mounted on a body contacting part to be placed on or around a region of interest of a user.

A user interface may be provided with a user input for activating an emergency mode, wherein the tightening device is adapted, upon activation of the emergency mode, to tighten the tightening system on or around a region of interest and adjust the tightening system in an emergency fastening position in which the pressure exerted by the device on the region of interest is at a maximum level or at least above systolic pressure, so that the tightening device acts as a tourniquet, in use.

In an aspect of the invention, the tightening system may comprise at least one of the following: DC motor, magnet, shape-memory alloy, manual winder, electromagnet, stepper motor, servomotor, and air pump.

The processor is adapted to cause the tightening of the tightening system on the region of interest, monitor the tightening pressure applied on the region of interest using the pressure sensor, process a sensor signal from the PPG or other sensor located downstream of the tightening system, to derive diastolic pressure and systolic pressure using both the pressure signal and the sensor signal. The tightening pressure exerted by the tightening system corresponds to the systolic pressure, whereas the PPG blood sensor cannot detect any variation of blood PPG signal.

According to the invention, the wearable blood pressure device comprises a reference sensor, located on the other side of the tightening system, with respect to the blood sensor, wherein, in use, the reference sensor is placed upstream of the tightening system and the blood sensor is placed downstream of the tightening system with respect to the blood flow of the arteries in the region of interest.

The processor is further adapted to compare the reference signal and the sensor signal to derive diastolic and systolic pressure.

The present invention also proposes a method of measuring blood pressure according to claim 6, comprising the following steps: causing tightening of a tightening system of a blood pressure device fastened on or around a region of interest, to increase pressure exerted by the tightening system on the region of interest, wherein the tightening is controlled by the processor using a pressure signal from a pressure sensor of the blood pressure device, the pressure sensor indicating the pressure exerted by the blood pressure device on the region of interest acquiring a blood signal by a blood sensor (e.g. PPG or other sensor) of the blood pressure device located downstream of the tightening system, and deriving the systolic pressure and the diastolic pressure using both the blood signal and the tightening pressure signal.

According to the invention, the method comprises acquiring a reference signal using a reference sensor placed upstream of the tightening system on the same region of interest as the blood sensor, comparing the reference signal with the blood signal, to derive the diastolic and/or the systolic pressure. The reference sensor is a PPG sensor.

The present invention also comprises the use of such a blood pressure measuring device in an ambulatory blood pressure monitoring (ABPM) device.

### Detailed description of the figures

These and other aspects of the invention will become apparent from, and elucidated with, reference to preferred embodiments described hereinafter with reference to the accompanying drawings, wherein:
Fig. 1 is a wearable tightening device according to an aspect of the disclosure;
Fig. 2 shows a tightening system of the tightening device according to one aspect of the disclosure;
Fig. 3 is a wearable blood pressure device according to an aspect of the disclosure;
Fig. 4 is the wearable blood pressure device of Fig. 3 in an operative position;
Fig. 5 is another wearable blood pressure device according to another aspect of the disclosure;
Figs. 6 and 7 show examples of blood signals using a method and/or a system of the present disclosure;
Fig. 8 shows a workflow of a method for measuring blood pressure according to one aspect of the disclosure.

### Detailed Description of the Invention

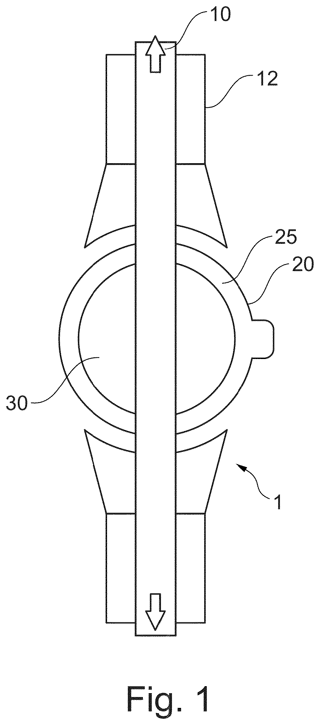

Fig. 1 shows a tightening device 1 to be placed around a region of interest of the user's body.

In one aspect of the invention, the tightening device is an auto-tightening device 1 and comprises a tightening system 10 for causing the auto-tightening device 1 to tighten on or around said region of interest, and a processor 20 to control the tightening system 10. In another aspect of the invention, the tightening device is controlled manually.

The tightening system 10 is provided for tightening and securing the tightening device 1 on or around the region of interest.

In the example of Fig. 1, a body contacting part 12 is provided, on both sides of the tightening system 10. This is an example only. There may be no additional body contacting part, or the tightening system 10 may be embedded within a body contacting part 12, placed on the body contacting part 12 or may be distinct from the body contacting part 12. In the example of Fig. 1, the tightening system 10 is distinct from the body contacting part 12.

The tightening system 10 is adapted to tighten the tightening device 1 in different required positions, as will be explained later in the disclosure. It should be understood that the tightening system 10 may also be adapted to tighten the body contacting part 12, when present.

The body contacting part 12 of the tightening device 1 and the tightening system 10 of Fig. 1 comprises a bracelet. The body contacting part/bracelet 12 may contain a thin layer of air inside. The bracelet is wrapped around the region of interest, e.g. an arm or thigh. This is not limiting the invention, and other types of body contacting parts 12 or tightening system 10 are possible, such as a band, a clamp, a cuff, a ring, a watch. These types depend on the shape of the region of interest, such as a wrist, a finger, an arm. For example, instead of a bracelet, one or more clamps and a ring could be provided to be placed around a fingertip.

The tightening device 1 may be incorporated into a smartwatch and placed around the wrist of the user's body, may be positioned around a thigh, or even adapted to be positioned on the limb with the tightening system adjusted at different tightening positions.

The tightening system 10 comprises at least one of the following: a small motor, a servomotor, an electromagnet pump or the like. It should be noted that the motor may include a gear train, and a control circuity 15.

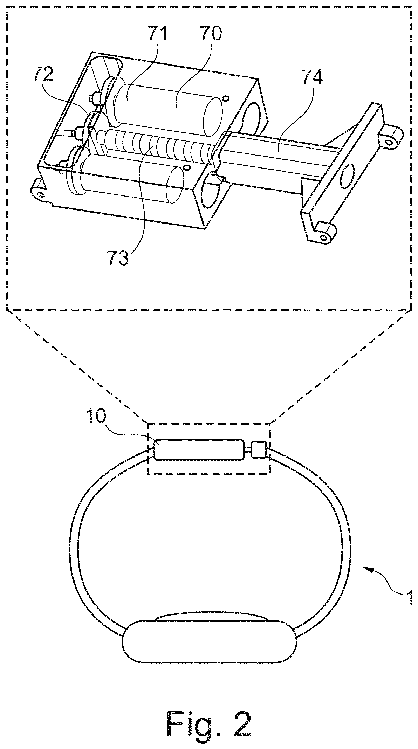

In the example of Fig. 2, the tightening device 1 comprises a transmission system with a motor 70 with a gear train 71 and a transmission 72, for rotating a male screw 73 interacting with a female screw 74. A redundant transmission system can be provided.

The tightening device 1 may also be activated manually, for example using a small winder. The tightening system 10 may provide the user with some information about the speed of the manual tightening that should be adopted for the tightening device 1, e.g. a proper speed to turn the winder.

The processor 20 is configured to receive and/or send control signals to the tightening system 10. A user interface 25 is provided on the tightening device 1 and allows interaction with the user wearing the tightening device 1.

The user interface 25 comprises a user input device 26, such as a microphone or a touch screen. The user input device 26 allows the user to enter control signals in order to select the position in which the bracelet 12 should be tightened, or the pressure exerted by the bracelet 12 on the region of interest.

The user interface 25 may also comprise an emergency button 28 activating an emergency function in the processor 20. When such an emergency function is activated, the processor 20 controls the tightening system to tighten the bracelet around the region of interest with as much pressure as possible, preferably at least above the systolic pressure of the subject wearing the tightening device 1, such that the bracelet forms a tourniquet to stop or limit a blood haemorrhage and thus to prevent a user from losing blood downstream from the tightening device 1, for example from the hand when the tightening device 1 is added in a smartwatch, from a thigh when the auto tightening device is wrapped around a thigh, or from the limbs when the tightening device 1 is positioned at the beginning of the limbs, i.e. the arms near the shoulders and the legs near the thighs. The emergency button can be operated by a user who is wounded and bleeding.

The user interface 25 may also comprise a display or a sound system for displaying or giving indications or information to the user.

It should be understood that such a tightening device 1 is adapted to block a haemorrhage when a subject is wounded. There are lots of different fields of application, such as the military, the police, firemen, and others involved in dangerous work. Of course, the tightening device 1 should be placed on the very beginning of the region of interest, e.g., to cover the arteries closest to the heart (such as limbs), or on other region of interest.

A pressure sensor 30 may also be provided on the tightening device 1. The pressure sensor 30 is preferably provided adjacent to or closed to the tightening system 10, on a body contacting part 12.

The pressure sensor 30 is adapted to measure the tightening pressure exerted by the tightening system 10 on the affected portion of the body. The pressure sensor 30 is adapted to be placed against the region of interest. The pressure sensor 30 may be a force sensor, strain sensor or a load cell.

The processor 20 is adapted to receive and process tightening pressure signals 30a representative of the force or pressure exerted by the bracelet from the pressure sensor 30. The pressure signals 30a are also used to control the tightening system 10 when tightening the tightening system 10 at different tightening positions.

The tightening device 1 may be used in military applications, for example for soldiers, or as police equipment. It can also be used for dangerous jobs applications, in which operators are exposed to risk of injury or to particular stressful or dangerous working conditions.

The tightening device 1 can measure the tightening pressure at predetermined time intervals. When a blood pressure drop is detected on the body part, the processor 20 can also activate the emergency function and control the tightening system 10 to tighten the bracelet 12 such as to apply a predetermined amount of pressure.

The tightening device 1 further includes one or more of the following: a battery, positioning capabilities, antenna capabilities such as RF or WIFI communications capabilities, *etc.* to allow remote monitoring and/or communications.

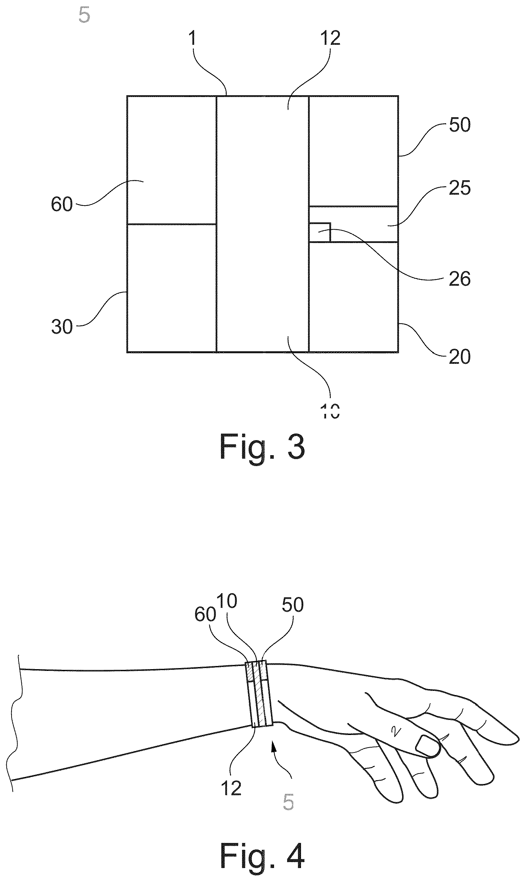

Fig. 3 shows an example of blood pressure device 5 according to an aspect of the present disclosure, and Fig. 4 shows the blood pressure device 5 in an operative position around a region of interest.

The blood pressure device 5 comprises the tightening device 1 having a pressure sensor 30 and a PPG sensor 50, mounted on the tightening device 1. According to examples not covered by the claimed invention, the PPG sensor 50 could be replaced by another type of blood sensor, such as but not limited to a piezoelectric sensor or an ultrasound sensor.

The tightening device 1 is similar to the device in Fig. 1 and comprises a body contacting part 12 for attaching the PPG sensor 50 to the region of interest, a tightening system 10 for tightening the tightening device 1 on or around the region of interest, and a processor 20.

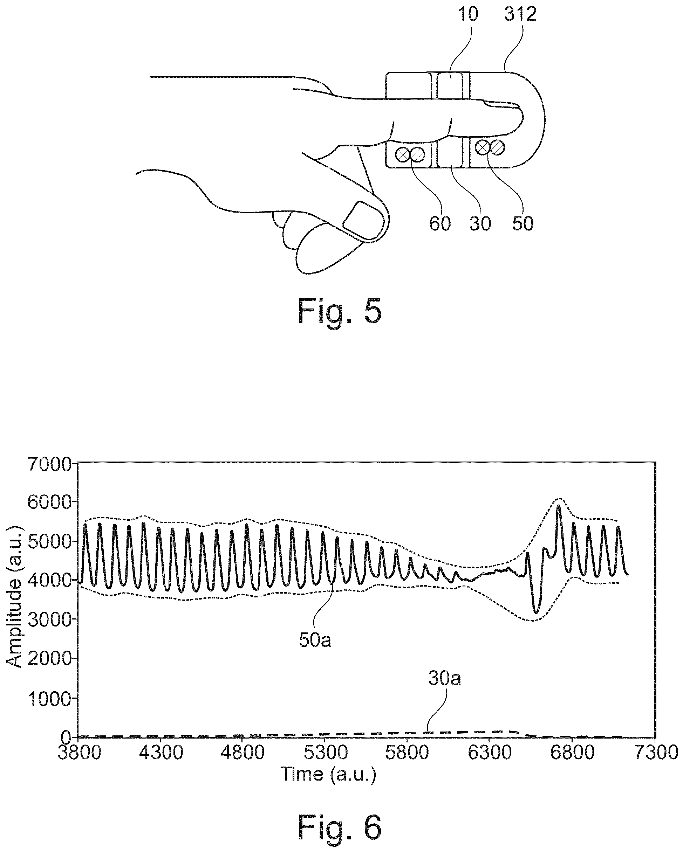

In the example of Figs. 3 and 4, the tightening device 1 is adapted to be wrapped around an arm or a thigh and therefore the body contacting part 12 is a bracelet or is incorporated into a bracelet, and the tightening system 10 also comprises a bracelet. This design is not limiting the invention and other body contacting parts for other types of tightening devices 1 are possible, depending on the shape of the region of interest. In the embodiment of Fig. 5, the tightening device 1 comprises a clamp 312 to be placed around a fingertip, instead of a bracelet 12 of Fig(s) 3 and 4.

The tightening system 10 is provided to tighten the region of interest in order to measure the blood pressure of the user. The tightening system 10, in one aspect of the disclosure, is adapted to automatically adjust the tightening system 10 in one of plurality of fastening positions. The plurality of fastening positions may correspond to a comfortable position, a running position, an acquisition position to acquire a good PPG signal, the automatic tightening mode when a pressure measurement is required, as well as the emergency position, in which the tightening system 10 is tightened and adjusted in a fastening position in which the pressure exerted by the blood pressure measurement device is at a maximum level, so that the blood pressure measurement device acts as a tourniquet. It would also be possible to use the tightening system as a tourniquet without the sensor 50 (or a reference sensor 60, which function is explained below).

The sensor 50 is a PPG sensor 50, which is located near the tightening system 10, mounted on the body contacting part 12 on one side of the tightening system 10. As is known in the art, the PPG sensor 50 is adapted to detect optically the light transmitted or reflected from or through tissues of the region of interest. The PPG sensor 50 detects changes in the blood flow volume by detecting changes in the detected light intensity. The PPG sensor 50 is able to measure volumetric changes and therefore the passage of blood into the vessels that, thanks to their elasticity, change their diameter every time there is a heartbeat.

The sensor 50 may be configured to work in a transmission or reflectance mode. The PPG sensors may use one or more wavelengths of light for their operation.

The blood pressure device 5 further comprises a reference sensor 60. The reference sensor 60 is adapted to be used as reference for the PPG sensor 50. The reference sensor 60 is located near the tightening system 10, mounted on the body contacting part 12 on other side of the tightening system 10 which is opposite the side of the sensor 50. Hence, the reference sensor 60 is configured to be located on the same artery, vessel or capillary bed, but not located far away from the sensor 50. The reference sensor 60 is also a PPG sensor.

In use, the blood pressure sensor device 5 is placed on the region of interest, for example with the bracelet 12 and the tightening system 10 around a forearm, with the sensor 50 located downstream of the tightening system 10, and the reference sensor 60 located upstream of the tightening system 10. The terms "downstream" and "upstream" are defined with respect to the direction of blood flow in the arteries in the human body. The tightening system 10 is therefore placed in between the sensors 50 and 60.

The pressure sensor 30 is adapted to measure the tightening pressure exerted by the tightening system 10 on the involved portion of the body. The pressure sensor 30 is adapted to be placed against or close to the region of interest.

The processor 20 is adapted to receive and process the tightening pressure signals 30a representative of the force or tightening pressure exerted by the tightening system 10 on the body. The tightening pressure signals 30a are supplied from the pressure sensor 30. The tightening pressure signals 30a may also be used to control the tightening system 10 when tightening the tightening system 10 at different tightening positions, as discussed above.

The processor 20 is configured to receive and/or send control signals to the tightening system 10. The processor further comprises data processing module(s) to process the different pressure signals, sensor signals, reference signals.

The user interface 25 may also comprise a display or a sound system for displaying or giving indications or information to the user, such as the value of applied pressure, the diastolic pressure, the systolic pressure, histograms of values, positions, locations, datum, or any even giving indication to the user on how to attach and operate the blood pressure device.

A blood pressure acquisition mode for measuring blood pressure is provided. The user may use the user interface 25 to select the blood pressure acquisition mode and start the procedure. In the blood pressure acquisition mode, the tightening system 10 is activated to automatically or manually tighten itself until the blood sensor 50 and the reference sensor 60 have detected the diastolic and systolic pressure. After detection of both the diastolic and systolic pressures, the tightening system 10 is adapted to release the pressure and/or force and return to the previous fastening position set before the start of the blood pressure acquisition procedure, in which the tightening system 10 is fixed around and/or on the region of interest.

The blood pressure device 5 further comprises a blood pressure monitoring mode, in which the blood pressure acquisition mode is activated at predetermined time intervals, which can be manually adjusted. An anomaly mode is also provided, in which the system is adapted to start a blood pressure measurement following an anomalous increase or decrease in the heart rate or heart rate variability detected by the pressure sensor 50 and/or the reference sensor 60.

The tightening system 10 can be adapted to tighten simultaneously the body contacting part 12 including the PPG sensor 50 and the reference sensor 60. However, the tightening system 10 may also be configured to tighten independently the body contacting part 12, the PPG sensor 50 and/or the reference sensor 60.

The pressure sensor 30 is configured to detect the tightening pressure applied by the blood pressure device 5 on the region of interest, in use.

The pressures sensor 30 may comprise one of the following: a piezoelectric sensor, piezoresistive sensor, inductive pressure sensor, capacitive or optical pressure sensor. The pressure exerted by the blood pressure device 5 may further be derived without the use of the pressure sensor 30. For example, when the tightening system 10 comprises a motor for tightening the tightening system 10, a current absorption of the motor of the tightening system may be used to derive the tightening pressure or force exerted by the blood pressure device on the region of interest. Finally, the pressure sensor could be replaced by a strain gauge or a load cell.

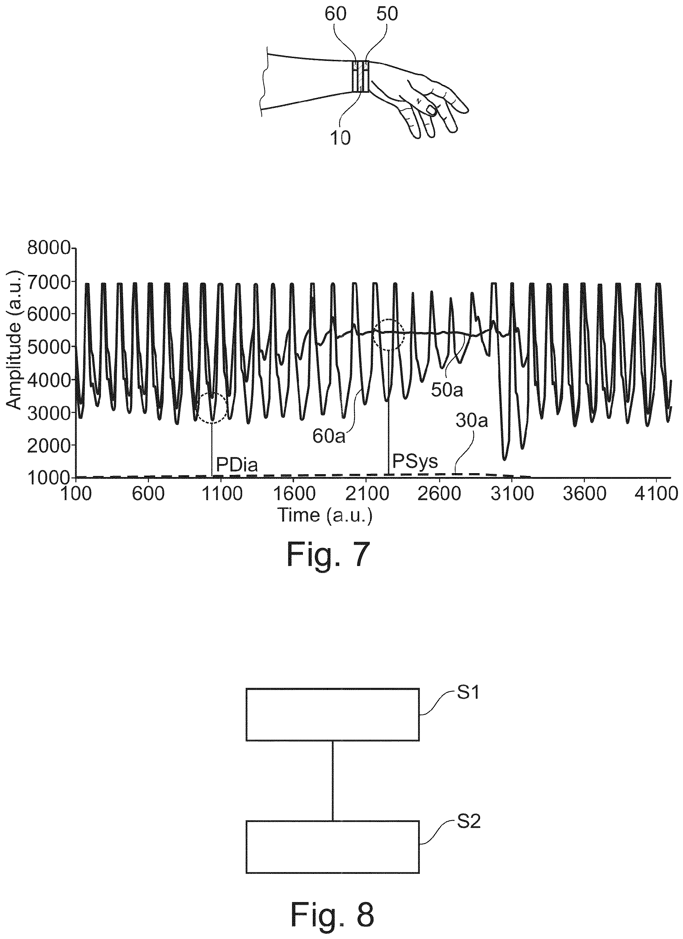

The blood pressure device 5 is adapted to measure the arterial blood pressure as explained in the following with reference to Fig(s) 6 and 7. Fig. 6 shows an example of a PPG signal (50a) acquired by the PPG sensor 50 and of a pressure signal 30a acquired by the pressure sensor 30. Fig. 7 shows an example of a blood PPG signal 50a and of a reference signal 60a acquired by the reference sensor 60 when the tightening pressure 30a exerted on the area of interest varies.

When a user, such as a subject, a patient or a paramedical staff, wants to measure the blood pressure, he/she can activate the blood pressure acquisition mode using the user interface 25. Otherwise, in a monitoring mode, blood pressure acquisition mode can be activated. In a first step S1, the tightening system 10 receives the control signal to tighten the tightening system 10, in order to increase the external pressure applied to the vessels of the area of interest, for example by means of the body contacting part 12 and/or tightening system 10.

The tightening pressure 30a is measured using the pressure sensor 30. The pressure sensor 30 reads the pressure value exerted by the tightening system 10 on the area of interest. The pressure value monitored by the pressure sensor 30 represents the external pressure exerted on the blood vessels.

The PPG sensor 50, which is located after the tightening system 10, detects a variation of the blood signal 50a caused by a diminished amount of blood in the blood vessel, when the external pressure increases. When the tightening pressure 30a applied by the body contacting part 12 and/or tightening system 10 reaches the systolic pressure, the blood vessel is completely obstructed, inhibiting the passage of blood downstream of the tightening system 10. The blood pressure sensor 50 is a PPG sensor 50, which is placed after the bracelet, cannot detect any variation of blood signal 50a. At this moment, the tightening pressure exerted by the tightening system 10 corresponds to the systolic pressure of the subject.

In a second step, the processor 20 is adapted to process the acquired measurements comprising the signal 50a and the tightening pressure 30a to derive the diastolic and systolic pressure (step S2). In particular, the processor processes an attenuation of the PPG signal 50a, associates the measured attenuations with the measured tightening pressure 30a applied by the tightening system and finally obtain the systolic and diastolic pressure.

The skilled person will understand that only the PPG sensor 50, together with the pressure sensor, can be used to determine both the systolic and diastolic pressure. In one embodiment, the blood pressure measurement device comprises only one PPG sensor.

According to the invention, the method comprises using the reference sensor 60, to improve the precision of the blood pressure measurement. In particular, the reference sensor 60, which is placed upstream of the tightening system 10, picks a signal from the same artery, arteriolar or capillary bed. As such, the reference sensor 60 can be used to improve the precision of the blood pressure measurement. In this embodiment, the processor 20 is adapted to process the acquired data comprising the signal 50a, the refence signal 60a and the tightening pressure 30a to derive the diastolic and systolic pressure.

When the tightening pressure exerted by the tightening system 10 on the area of interest increases, the reference sensor 60 does not determine or only partially determine a decrease of the reference signal 60a, since the tightening system 10 blocks the circulation of the blood only in those blood vessels that are located downstream of the bracelet 10.

The processor 20 uses the reference signal 60a from the refence sensor 60, and is adapted to compare the amplitude variation of the reference signal 60a and of the PPG signal 50a, to derive information on the patient's diastolic and systolic pressure. The reference signal 60a captured by the reference sensor 60 is used to identify when the sensor signal 50a begins to decrease in the PPG sensor 50, i.e. to identify the diastolic pressure value.

The blood pressure measuring device may be part of an ambulatory blood pressure measurement (ABPM) device, a sphygmomanometer, a pulse oximeter or a smartwatch.

While the present disclosure has been described with reference to blood pressure measurement, it should be noted that of course PPG sensor may additionally be used to measure heart rate and blood oxygenation, as well as a plurality of abnormalities in the morphology of the heart signal.

## Claims

1. A wearable arterial blood pressure measuring device (1) for measuring at least one of the systolic pressure and, the diastolic pressure of a user, the blood pressure measuring device comprising:
a tightening system (10) with a tightening device adapted to tighten the tightening device on or around a region of interest of the user, wherein the tightening system (10) is adapted to be adjusted in one of a plurality of fastening positions;
a blood sensor (50) placed on one side of the tightening system (10);
one of a processor (20) or control circuitry (12) to control the tightening system (10);and
a pressure sensor (30) for measuring the tightening pressure (30a) exerted by the tightening system (10) on the region of interest, in particular wherein the pressure sensor (30) is mounted on a body contacting part (12) to be placed on or around a region of interest of a user,
a reference sensor (60), located on the other side of the tightening system (10) with respect to the blood sensor (50),
wherein
the blood sensor (50) is a PPG sensor (50) placed downstream of the tightening system (10) with respect to the blood flow of the arteries in the region of interest outputting a PPG signal (50a), and the reference sensor (60) is a PPG sensor placed upstream of the tightening system (10),
the processor (20) is adapted to cause the tightening of the tightening system (10) on the region of interest, monitor the tightening pressure (30a) applied on the region of interest using the pressure sensor (30), process said PPG signal (50a) from the PPG sensor (50) located downstream of the tightening system (10), to derive the systolic pressure and the diastolic pressure using both the tightening pressure signal (30a) and the PPG signal (50a), wherein, when the PPG blood sensor (50) cannot detect any variation of blood PPG signal (50a), the tightening pressure (30a) exerted by thetightening system (10) corresponds to the systolic pressure, and
wherein the processor (20) is further adapted to compare the reference signal (60a) and the PPG signal (50a) to derive the diastolic pressure and the systolic pressure, and is adapted to compare the amplitude variation of the reference signal (60a) and of the PPG signal (50a), wherein the reference signal (60a) captured by the reference sensor (60) is used to identify when the sensor PPG signal (50a) begins to decrease in the PPG blood sensor (50) and hence to identify the diastolic pressure value and the systolic pressure value.

2. The device of claim 1, comprising a user interface with a user input provided for activating an emergency mode, wherein the tightening device (1) is adapted, upon activation of the emergency mode, to tighten the tightening system (10) on or around a region of interest and adjust the tightening system (10) in an emergency fastening position in which the pressure exerted by the tightening device (1) on the region of interest is at a maximum level, so that the tightening device (1) acts as a tourniquet, in use.

3. The device of claim 1 or 2, wherein the tightening system (10) comprises at least one of the following: DC motor, magnet, electromagnet, stepper motor, servomotor, air pump.

4. A method of measuring blood pressure using a wearable arterial blood pressure measuring device (1), comprising the following steps:
causing tightening of a tightening system (10) of the blood pressure measuring device (10) fastened on or around a region of interest, to increase pressure exerted by the tightening system (10) on the region of interest, wherein the tightening is controlled by a processor (20) or a control circuitry (12) using a pressure signal (30a) from a pressure sensor (30) of the blood pressure measuring device (10),
the pressure sensor (30) indicating the pressure exerted by the blood pressure device on the region of interest;
acquiring a PPG signal (50a) by a blood sensor (50) of the blood pressure device located downstream of tightening system and a reference signal (60a) using a reference sensor (60) placed upstream of the tightening system (10) on the same region of interest as the blood sensor (50)
comparing the reference signal (60a) with the blood PPG signal (50a), to derive the diastolic and the systolic pressure, wherein the comparison comprises comparing the amplitude variation of the reference signal (60a) and of the PPG signal (50a),
deriving the systolic pressure and the diastolic pressure using both the PPG signal (50a) and the tightening pressure signal (30a), wherein, when the blood sensor (50) cannot detect any variation of PPG signal (50a), the tightening pressure exerted by the tightening system corresponds to the systolic pressure, and
wherein the reference signal (60a) captured by the reference sensor (60) is used to identify when the PPG signal (50a) begins to decrease in the PPG blood sensor (50),
wherein the blood sensor (50) is a PPG sensor (50) outputting a PPG signal (50a) and the reference sensor (60) is a PPG sensor.

5. Use of the wearable blood pressure measuring in device according to any of claims 1 to 4 in one of an ambulatory blood pressure measurement device, a sphygmomanometer, a pulse oximeter, or a smartwatch.

## Patentansprüche

1. Tragbare arterielle Blutdruckmessvorrichtung (1) zum Messen von mindestens einem von dem systolischen Blutdruck und dem diastolischen Blutdruck eines Benutzers, wobei die Blutdruckmessvorrichtung umfasst:
ein Festziehsystem (10) mit einer Festziehvorrichtung, die zum Festziehen der Festziehvorrichtung auf einer oder um eine Interessensregion des Benutzers vorgesehen ist, wobei das Festziehsystem (10) vorgesehen ist, um in eine von einer Vielzahl von Festziehpositionen eingestellt zu werden;
einen Blutsensor (50), der auf einer Seite des Festziehsystems (10) platziert ist;
einen von einem Prozessor (20) oder einer Steuerschaltanordnung (12), um das Festziehsystem (10) zu steuern; und
einen Drucksensor (30) zum Messen des Festziehdrucks (30a), der durch das Festziehsystem (10) auf die Interessensregion ausgeübt wird, wobei der Drucksensor (30) insbesondere auf einem Körperkontaktteil (12) angebracht ist, das auf einer oder um eine Interessensregion eines Benutzers zu platzieren ist,
einen Referenzsensor (60), der sich auf der anderen Seite des Festziehsystems (10) in Bezug auf den Blutsensor (50) befindet,
wobei
der Blutsensor (50) ein PPG-Sensor (50) ist, der in Bezug auf den Blutfluss der Arterien in der Interessensregion nachgeordnet zu dem Festziehsystem (10) platziert ist und ein PPG-Signal (50a) ausgibt, und der Referenzsensor (60) ein PPG-Sensor ist, der vorgeordnet zu dem Festziehsystem (10) platziert ist,
wobei der Prozessor (20) vorgesehen ist, um das Festziehen des Festziehsystems (10) auf der Interessensregion zu veranlassen, den auf die Interessensregion ausgeübten Festziehdruck (30a) unter Verwendung des Drucksensors (30) zu überwachen, das PPG-Signal (50a) von dem PPG-Sensor (50) zu verarbeiten, der sich nachgeordnet zu dem Festziehsystem (10) befindet, um den systolischen Druck und den diastolischen Druck unter Verwendung von sowohl dem Festziehdrucksignal (30a) als auch dem PPG-Signal (50a) abzuleiten, wobei, wenn der PPG-Blutsensor (50) keinerlei Variation des Blut-PPG-Signals (50a) detektieren kann, der durch das Festziehsystem (10) ausgeübte Festziehdruck (30a) dem systolischen Druck entspricht, und
wobei der Prozessor (20) des Weiteren vorgesehen ist, um das Referenzsignal (60a) und das PPG-Signal (50a) zu vergleichen, um den diastolischen Druck und den systolischen Druck abzuleiten, und vorgesehen ist, um die Amplitudenvariation des Referenzsignals (60a) und des PPG-Signals (50a) zu vergleichen, wobei das durch den Referenzsensor (60) aufgenommene Referenzsignal (60a) verwendet wird, um zu identifizieren, wann das Sensor-PPG-Signal (50a) in dem PPG-Blutsensor (50) abzunehmen beginnt, und dadurch den diastolischen Druckwert und den systolischen Druckwert zu identifizieren.

2. Vorrichtung nach Anspruch 1, umfassend eine Benutzerschnittstelle, bei der eine Benutzereingabe zum Aktivieren eines Notfallmodus bereitgestellt ist, wobei die Festziehvorrichtung (1) vorgesehen ist, um bei Aktivierung des Notfallmodus das Festziehsystem (10) auf einer oder um eine Interessensregion festzuziehen und das Festziehsystem (10) in eine Notfallbefestigungsposition einzustellen, in der der durch die Festziehvorrichtung (1) auf die Interessensregion ausgeübte Druck sich auf einem maximalen Niveau befindet, so dass die Festziehvorrichtung (1) bei Gebrauch als Tourniquet wirkt.

3. Vorrichtung nach Anspruch 1 oder 2, wobei das Festziehsystem (10) mindestens eines der Folgenden umfasst: Gleichstrommotor, Magnet, Elektromagnet, Schrittmotor, Servomotor, Luftpumpe.

4. Verfahren zum Messen des Blutdrucks unter Verwendung einer tragbaren arteriellen Blutdruckmessvorrichtung (1), umfassend die folgenden Schritte:
Veranlassen von Festziehen eines Festziehsystems (10) der Blutdruckmessvorrichtung (10), die auf einer oder um eine Interessensregion befestigt ist, um den durch das Festziehsystem (10) auf die Interessensregion ausgeübten Druck zu erhöhen, wobei das Festziehen durch einen Prozessor (20) oder eine Steuerschaltungsanordnung (12) unter Verwendung eines Drucksignals (30a) von einem Drucksensor (30) der Blutdruckmessvorrichtung (10) gesteuert wird,
wobei der Drucksensor (30) den durch die Blutdruckvorrichtung auf die Interessensregion ausgeübten Druck angibt;
Erfassen eines PPG-Signals (50a) durch einen Blutsensor (50) der Blutdruckvorrichtung, der sich nachgeordnet zu dem Festziehsystem befindet, und eines Referenzsignals (60a) unter Verwendung eines Referenzsensors (60), der vorgeordnet zu dem Festziehsystem (10) auf derselben Interessensregion wie der Blutsensor (50) platziert ist,
Vergleichen des Referenzsignals (60a) mit dem Blut-PPG-Signal, um den diastolischen und den systolischen Druck abzuleiten, wobei der Vergleich Vergleichen der Amplitudenvariation des Referenzsignals (60a) und des PPG-Signals (50a) umfasst,
Ableiten des systolischen Drucks und des diastolischen Drucks unter Verwendung von sowohl dem PPG-Signal (50a) als auch dem Festziehdrucksignal (30a), wobei, wenn der Blutsensor (50) keinerlei Variation des PPG-Signals (50a) detektieren kann, der durch das Festziehsystem ausgeübte Festziehdruck dem systolischen Druck entspricht, und
wobei das durch den Referenzsensor (60) aufgenommene Referenzsignal (60a) verwendet wird, um zu identifizieren, wann das PPG-Signal (50a) in dem PPG-Blutsensor (50) abzunehmen beginnt,
wobei der Blutsensor (50) ein PPG-Sensor (50) ist, der ein PPG-Signal (50a) ausgibt, und der Referenzsensor (60) ein PPG-Sensor ist.

5. Verwendung der tragbaren Blutdruckmessvorrichtung nach einem der Ansprüche 1 bis 4 in einer von einer ambulanten Blutdruckmessvorrichtung, einem Sphygmomanometer, einem Pulsoximeter oder einer Smartwatch.

## Revendications

1. Dispositif (1) portable de mesure de la tension artérielle destiné à mesurer au moins une tension parmi la tension systolique et la tension diastolique d'un utilisateur, le dispositif de mesure de la tension artérielle comprenant :
un système de serrage (10) pourvu d'un dispositif de serrage conçu pour serrer le dispositif de serrage sur ou autour d'une région d'intérêt de l'utilisateur, le système de serrage (10) étant conçu pour être ajusté dans une position parmi une pluralité de positions de fixation ;
un capteur sanguin (50) placé d'un côté du système de serrage (10) ;
un élément parmi un processeur (20) ou un circuit de commande (12), destiné à commander le système de serrage (10) ; et
un capteur de pression (30) destiné à mesurer la pression de serrage (30a) exercée par le système de serrage (10) sur la région d'intérêt, en particulier le capteur de pression (30) étant monté sur une partie de contact (12) avec le corps à placer sur ou autour d'une région d'intérêt d'un utilisateur,
un capteur de référence (60), situé de l'autre côté du système de serrage (10) par rapport au capteur sanguin (50),
dans lequel
le capteur sanguin (50) est un capteur PPG (50) placé en aval du système de serrage (10) par rapport au flux sanguin des artères dans la région d'intérêt et délivre un signal PPG (50a) et le capteur de référence (60) est un capteur PPG placé en amont du système de serrage (10),
le processeur (20) est conçu pour provoquer le serrage du système de serrage (10) sur la région d'intérêt, pour surveiller la pression de serrage (30a) appliquée sur la région d'intérêt à l'aide du capteur de pression (30), pour traiter ledit signal PPG (50a) provenant du capteur PPG (50) situé en aval du système de serrage (10), afin de déduire la tension systolique et la tension diastolique à l'aide à la fois du signal de pression de serrage (30a) et du signal PPG (50a), dans lequel, lorsque le capteur sanguin PPG (50) ne peut détecter aucune variation du signal PPG sanguin (50a), la pression de serrage (30a) exercée par le système de serrage (10) correspond à la tension systolique et
dans lequel le processeur (20) est en outre conçu pour comparer le signal de référence (60a) au signal PPG (50a) afin de déduire la tension diastolique et la tension systolique et est conçu pour comparer la variation d'amplitude du signal de référence (60a) et du signal PPG (50a), le signal de référence (60a) capturé par le capteur de référence (60) étant utilisé pour identifier le moment où le signal PPG (50a) de capteur commence à diminuer dans le capteur sanguin PPG (50) et donc pour identifier la valeur de tension diastolique et la valeur de tension systolique.

2. Dispositif selon la revendication 1, comprenant une interface utilisateur pourvue d'une entrée utilisateur prévue pour activer un mode d'urgence, dans lequel le dispositif de serrage (1) est conçu, lors de l'activation du mode d'urgence, pour serrer le système de serrage (10) sur ou autour d'une région d'intérêt et pour ajuster le système de serrage (10) dans une position de fixation d'urgence dans laquelle la pression exercée par le dispositif de serrage (1) sur la région d'intérêt est à un niveau maximal, de telle sorte que le dispositif de serrage (1) agit comme un garrot, lors de l'utilisation.

3. Dispositif selon la revendication 1 ou 2, dans lequel le système de serrage (10) comprend au moins l'un des éléments suivants : moteur à courant continu, aimant, électroaimant, moteur pas à pas, servomoteur, pompe à air.

4. Procédé de mesure de la tension artérielle à l'aide d'un dispositif (1) portable de mesure de la tension artérielle, comprenant les étapes suivantes :
provocation du serrage d'un système de serrage (10) du dispositif (10) de mesure de la tension artérielle fixé sur ou autour d'une région d'intérêt, afin d'augmenter la pression exercée par le système de serrage (10) sur la région d'intérêt, le serrage étant commandé par un processeur (20) ou un circuit de commande (12) à l'aide d'un signal de pression (30a) provenant d'un capteur de pression (30) du dispositif (10) de mesure de la tension artérielle,
le capteur de pression (30) indiquant la pression exercée par le dispositif de tension artérielle sur la région d'intérêt ;
acquisition d'un signal PPG (50a) par un capteur sanguin (50) du dispositif de tension artérielle situé en aval du système de serrage et d'un signal de référence (60a) à l'aide d'un capteur de référence (60) placé en amont du système de serrage (10) sur la même région d'intérêt que le capteur sanguin (50) ;
comparaison du signal de référence (60a) au signal PPG sanguin (50a), afin de déduire la tension diastolique et la tension systolique, la comparaison comprenant la comparaison de la variation d'amplitude du signal de référence (60a) et du signal PPG (50a) ;
déduction de la tension systolique et de la tension diastolique à l'aide à la fois du signal PPG (50a) et du signal de pression de serrage (30a), dans lequel, lorsque le capteur sanguin (50) ne peut détecter aucune variation du signal PPG (50a), la pression de serrage exercée par le système de serrage correspond à la tension systolique et
dans lequel le signal de référence (60a) capturé par le capteur de référence (60) est utilisé pour identifier le moment où signal PPG (50a) commence à diminuer dans le capteur sanguin PPG (50),
dans lequel le capteur sanguin (50) est un capteur PPG (50) délivrant un signal PPG (50a) et le capteur de référence (60) est un capteur PPG.

5. Utilisation du dispositif portable de mesure de la tension artérielle selon l'une quelconque des revendications 1 à 4 dans un dispositif parmi un dispositif ambulatoire de mesure de la tension artérielle, un sphygmomanomètre, un oxymètre de pouls ou une montre intelligente.
